# Europäisches Patentamt
# European Patent Office
# Office européen des brevets

(19)

(11) Veröffentlichungsnummer : **0 146 772 B1**

(12) ## EUROPÄISCHE PATENTSCHRIFT

(45) Veröffentlichungstag der Patentschrift :
10.02.88

(21) Anmeldenummer : 84113913.2

(22) Anmeldetag : 16.11.84

(51) Int. Cl.⁴ : **A 61 M 5/16**

(54) **Vorrichtung zur Registrierung der Tropfen in einem Infusionsgerät.**

(30) Priorität : 09.12.83 DE 3344632

(43) Veröffentlichungstag der Anmeldung :
03.07.85 Patentblatt 85/27

(45) Bekanntmachung des Hinweises auf die Patenterteilung : 10.02.88 Patentblatt 88/06

(84) Benannte Vertragsstaaten :
CH DE FR GB IT LI SE

(56) Entgegenhaltungen :
GB-A- 1 331 432
GB-A- 1 560 243
GB-A- 2 007 354
US-A- 3 700 904
IEEE TRANSACTIONS ON COMPUTERS, Band C-22, Nr. 2 Februar 1973, Seiten 168-171, S.L. HIGHT et al.: "Dissent in a majority voting system"

(73) Patentinhaber : Siemens-Elema AB
Röntgenvägen 2
S-171 95 Solna 1 (SE)
SE
Siemens Aktiengesellschaft
Berlin und München Wittelsbacherplatz 2
D-8000 München 2 (DE)
CH DE FR GB IT LI

(72) Erfinder : Cewers, Göran
Moellevongsvaegen 89
S-222 40 Lund (SE)
Erfinder : Olsson, Sven-Gunnar, Dipl.-Ing.
Postlada 652
S-240 17 Soedra Sandby (SE)

## Beschreibung

Die Erfindung betrifft eine Vorrichtung zur Registrierung der Tropfen in einem Infusionsgerät mit einer an einen Vorratsbehälter für Infusionsflüssigkeit angeschlossenen Tropfenkammer, von der die Infusionsflüssigkeit über eine Leitung einem Patienten zuführbar ist, und mit einem im Bereich der Tropfenkammer vorgesehenen Tropfenfühler, der mindestens eine Lichtquelle und zumindest zwei in Tropfenrichtung versetzt angeordnete Lichtsensoren aufweist (US-A-3 700 904).

Verschiedene Faktoren können die Registrierung eines Tropfens in einer Tropfenkammer erschweren, wenn optische Messmethoden angewendet werden. So kann die Transparenz der Wand der Tropfenkammer verschlechtert werden, wenn sich z. B. Flüssigkeitsdampf darauf niederschlägt oder wenn diese mit Spritzern benetzt wird, die beim Auftreffen eines Tropfens auf die Flüssigkeitsoberfläche oder durch Erschütterungen der Tropfenkammer entstehen können. Derartige Spritzer oder eine durch Erschütterungen verursachte wellige Flüssigkeitsoberfläche können darüberhinaus falsche Tropfenanzeigen auslösen. Vibrationen oder eine Neigung der Tropfenkammer können weiterhin dazu führen, dass der Tropfen schräg durch die Tropfenkammer fällt. Ebenso ist es auch möglich, dass die für die Registrierung verwendete Elektronik, die Lichtquelle oder der Sensor mit der Zeit in ihren Eigenschaften nachlassen und dadurch die Registrierung unsicherer wird.

Zur Vermeidung dieser möglichen Fehler bei der Tropfenregistrierung ist beispielsweise aus der DE-OS 28 30 512 eine Vorrichtung bekannt, bei der mehrere Lichtquellen und Fotodetektoren auf dem Umfang der Tropfenkammer in einer Ebene senkrecht zur Tropfenrichtung angeordnet sind. Dadurch soll zumindest erreicht werden, dass auch bei ungünstiger Schräglage der Tropfenkammer noch eine einwandfreie Tropfenregistrierung möglich ist.

Weiterhin ist aus der US-PS 40 38 981 eine Anordnung bekannt, bei der in einer Ebene eine Lichtquelle und zwei Fototransistoren angeordnet sind. Auch hierdurch soll die Empfindlichkeit des Tropfenfühlers erhöht und Schräglagen berücksichtigt werden. Dabei wird eine Flüssigkeitsleitung über einen Taktgeber freigegeben und gleichzeitig ein Fehlerglied gesetzt, das — wenn innerhalb einer vorbestimmten Zeit vom Tropfendetektor kein Rücksetzsignal kommt — eine Fehleranzeige und/oder einen Alarm auslöst. Bei dieser Anordnung muss jedesmal durch Betätigen eines Ventiles ein Tropfen freigegeben werden, dessen Auftreten dann über den Tropfenfühler nachgewiesen wird, wodurch dann gleichzeitig das Ventil wieder geschlossen wird.

Die Fototransistoren sind parallel geschaltet. Ihre Kollektorspannungen sind wechselspannungsmässig auf den nachfolgenden Impulsformer geschaltet. Dadurch wird die Schaltung auch dann, wenn weniger Licht auf die Fototransistoren fällt, ausbalanciert. Wird jedoch die Empfindlichkeit der Anordnung aus irgendeinem Grunde so schlecht, dass die Tropfendetektion unsicher ist, so kann das zu schwerwiegenden Fehlern führen. So kann es z. B. vorkommen, dass nur jeder zweite Tropfen registriert wird und einem Patienten dadurch eine unzulässig grosse Menge an Infusionsflüssigkeit zugeführt wird. Auch kann z. B. eine Blutdruckmessung die Tropfenauslösung verzögern und völlig unerwünscht über die Fehlerstufe einen Alarm auslösen.

Der vorliegenden Erfindung liegt die Aufgabe zugrunde, eine Vorrichtung der eingangs genannten Art anzugeben, bei der auf einfache Weise und ohne notwendige Kopplung mit einer Ventilanordnung zur Tropfenfreigabe Fehler in der Tropfenregistrierung vermieden werden können und bei der eine einfache Funktionskontrolle der Tropfenfühler möglich ist.

Diese Aufgabe wird erfindungsgemäss durch die im kennzeichnenden Teil des Anspruches 1 angegebenen Merkmale gelöst. Der Tropfenfühler ist in mindestens zwei Ebenen aufgeteilt, von der jede seine eigene Kette von Detektorschaltung mit beispielsweise Verstärker, Komparator und Speicherglied aufweist. Die Ausgangssignale jeder Kette sind auf eine gemeinsame Logikstufe geschaltet, die beim Vorliegen eines Signales von mindestens einer Kette, insbesondere aber von allen Ketten, eine Tropfenanzeige ansteuern kann. Die Ausgangssignale der Komparatoren sind weiterhin auf eine Diskriminatorstufe geschaltet, die auch ein Signal von der Logikstufe erhält. Die Diskriminatorstufe arbeitet dabei derart, dass — wenn innerhalb einer bestimmten Zeit nach dem Auftreten eines ersten Komparatorsignales kein Signal von der Logikstufe kommt, das anzeigt, dass alle Ebenen den Tropfen detektiert haben — eine Fehlanzeige aktiviert wird. Die Diskriminatorstufe zeigt damit an, wenn die Detektierung eines Tropfens durch die Tropfenfühler und die nachfolgende Detektorschaltungen aus irgendeinem Grunde unsicher wird.

Auch bei der erfindungsgemässen Vorrichtung ist vorteilhaft vorgesehen, dass die Ausgangssignale der einzelnen Tropfenfühler, gegebenenfalls nach Verstärkung, wechselspannungsmässig auf die nachfolgenden Glieder, wie z. B. Impulsformer oder Komparator geschaltet sind. Dadurch ist auch hier innerhalb bestimmter Grenzen eine von der Transparenz der Tropfenkammer unabhängige Tropfenregistrierung möglich. Im Gegensatz zu den bekannten Vorrichtungen ist es mit der erfindungsgemässen Vorrichtung aufgrund der in mindestens zwei Ebenen angeordneten Tropfenfühler möglich, die Funktionssicherheit der gesamten Vorrichtung zu überprüfen. Besonders vorteilhaft kann dazu vorgesehen sein, dass jede Ebene eine andere Empfindlichkeit aufweist. Da ein Fehlersignal ausgelöst wird, wenn nicht alle Ebenen innerhalb einer vorgegebenen Zeit einen Tropfen detektiert haben, kann damit im entscheidenden Uebergangsbereich, in dem die Tropfendetektion unsicher ist, rechtzeitig ein Fehler- und Warnsignal erzeugt werden.

Der Energieverbrauch der Vorrichtung wird in Weiterbildung der Erfindung vorteilhaft dadurch gesenkt, dass sowohl die Lichtquelle als auch die Lichtsensoren getaktet sind. Um eine sichere Weiterverarbeitung der gepulsten Signale zu gewährleisten, kann vorteilhafter Weise ein « sample and hold »-Glied in der Detektorschaltung vorgesehen sein, das ebenfalls und synchron zu Lichtquelle und Lichtsensor getaktet ist.

Um auf einfache Weise schnell eine falsche Lage der Tropfenkammer anzeigen zu können, ist in Weiterbildung der Erfindung vorgesehen, dass die Tropfenkammer mit einem Magneten versehen ist und eine Haltevorrichtung, in der die Tropfenkammer angebracht ist, an entsprechender Stelle mit einem Hall-Element. Das Ausgangssignal des Hall-Elementes ist auf ein Speicher-Flipflop geschaltet. Befindet sich die Tropfenkammer nicht in der vorgeschriebenen Lage, so ist der Abstand zwischen Magnet und Hall-Element zu gross und es ergibt sich ein Ausgangssignal am Hall-Element, das das Speicher-Flipflop setzt und damit den Zähler startet. Da die anderen Speicher-Flipflops nicht gesetzt werden, wird nach der vorgegebenen Zählerzeit ein Fehlersignal erzeugt. Auch hier ist es vorteilhaft zur Senkung des Energieverbrauches, wenn das Hall-Element getaktet ist.

Weitere Einzelheiten und Vorteile der Erfindung ergeben sich aus den Unteransprüchen und aus dem anhand von drei Figuren beschriebenen Ausführungsbeispiel. Dabei zeigt

Fig. 1 schematisch den Aufbau eines Infusionsgerätes mit daran angeschlossener Vorrichtung zur Registrierung der Tropfen

Fig. 2 in einem vereinfachten Blockschaltbild den prinzipiellen Aufbau dieser Vorrichtung und

Fig. 3 eine mögliche Schaltungsausführung.

Bei dem anhand der nachfolgend beschriebenen Figuren dargestellten Ausführungsbeispiel weist der Tropfenfühler zwei Ebenen auf. Im Rahmen der vorliegenden Erfindung ist es jedoch auch möglich, weitere Ebenen vorzusehen.

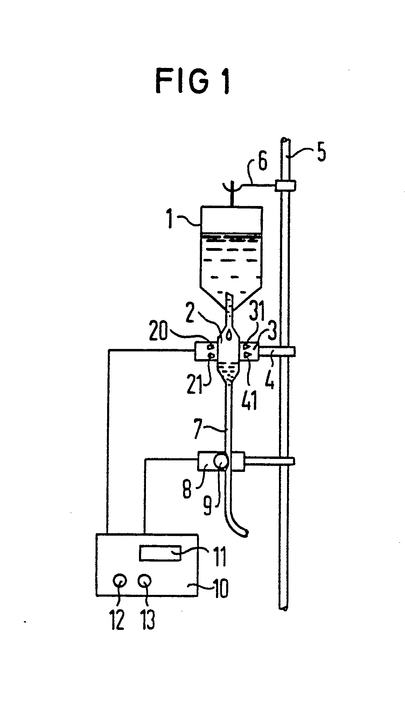

In Fig. 1 ist mit 1 eine Flasche für Infusionsflüssigkeit bezeichnet, an die in üblicher Weise eine Tropfenkammer 2 angeschlossen ist. Die Tropfenkammer besteht aus transparentem Material, so dass der Tropfenvorgang beobachtet werden kann. Die Tropfenkammer ist in einer Halterung 3 befestigt, die über ein Verbindungsstück 4 an einem Stativ 5 angebracht ist. Angedeutet ist weiterhin ein Bügel 6, der ebenfalls am Stativ 5 angebracht ist und der zum Halten der Flasche dient.

An die Tropfenkammer 2 ist ein Schlauch 7 angeschlossen, über den die Infusionsflüssigkeit einem Patienten zugeführt werden kann. Ueber eine Klemmvorrichtung 8 mit einem Exzenter 9, die ebenfalls an dem Stativ angebracht sein kann, kann der Schlauchquerschnitt und damit die Durchflussmenge reguliert werden.

Schliesslich ist ein Anzeige- und Steuergerät 10 angedeutet, das sowohl mit der Klemmvorrichtung 8 als auch dem Tropfenfühler verbunden ist. Ueber dieses Anzeige- und Steuergerät 10 kann beispielsweise die gewünschte Durchflussmenge, d. h. die Tropfenzahl, oder auch die total zuzuführende Infusionsflüssigkeitsmenge eingestellt und kontrolliert werden. Ueber eine Anzeige 11 können beispielsweise die momentane Tropfengeschwindigkeit oder die bereits zugeführte Infusionsflüssigkeitsmenge oder ähnliche Daten dargestellt werden. Durch eine Lampe 12 oder entsprechende Leuchtdioden können beispielsweise die einzelnen Tropfen optisch angezeigt werden. Eine weitere optische Anzeige 13 kann beispielsweise einen Fehler in der Anordnung anzeigen. Selbstverständlich kann im Fehlerfalle auch in bekannter Weise ein akustischer Alarm ausgelöst werden und/oder die Flüssigkeitszufuhr über die Klemmvorrichtung unterbrochen werden.

Die Haltevorrichtung 3 enthält die erfindungsgemässe Vorrichtung zur Registrierung der Tropfen mit zwei Lichtquellen 20 und 21, zwei Sensoren 31 und 41 und zusätzlich ein hier nicht dargestelltes Hall-Element. Im Bereich des Hall Elementes muss die Tropfenkammer dann mit einem Magneten versehen sein. Beide sind der Uebersichtlichkeit halber in der schematischen Fig. 1 nicht näher dargestellt.

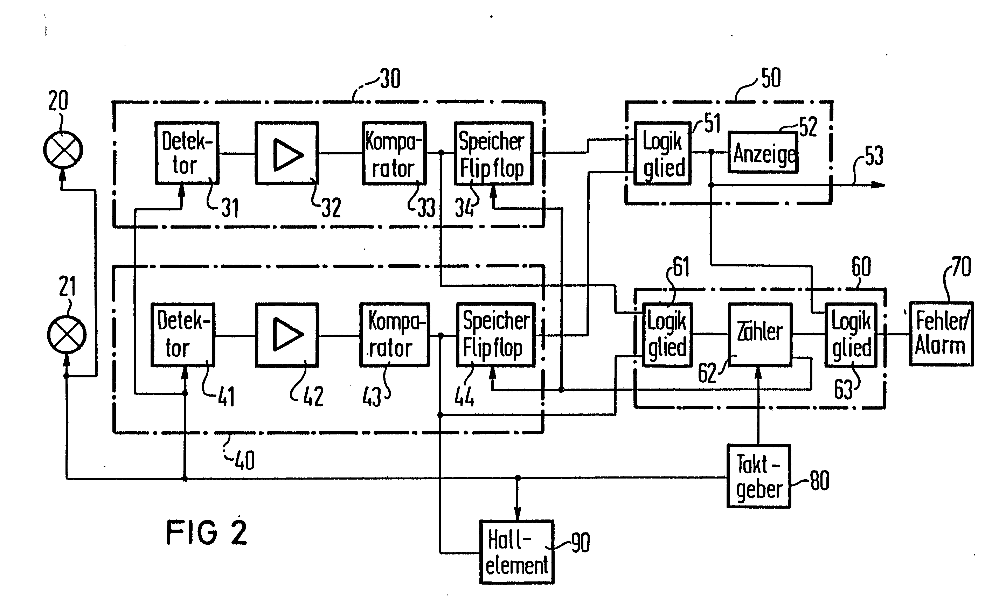

In der folgenden Fig. 2 ist die Vorrichtung zur Registrierung der Tropfen in einem Blockschaltbild dargestellt. Sie enthält zwei Lichtquellen 20 und 21, die in Tropfenrichtung gesehen hintereinander in der Haltevorrichtung 3 gemäss Fig. 1 angeordnet sind. Als Lichtquellen können beispielsweise Leuchtdioden dienen. Ebenso ist es jedoch möglich, nur eine einzige Lichtquelle vorzusehen und das davon ausgehende Lichtbündel mit Hilfe von Linsen so aufzuweiten, dass ein grösserer Bereich der Tropfenkammer durchleuchtet wird.

Gegenüber der Lichtquelle oder den Lichtquellen sind zwei Tropfenfühler 30 bzw. 40 angeordnet. Diese bestehen im vorliegenden Ausführungsbeispiel jeweils aus einem Detektor 31 bzw. 41, beispielsweise einem Fotodiode, einem Verstärker 32 bzw. 42, einem Komparator 33 bzw. 43 und einem Speicher 34 bzw. 44. Die Ausgänge der beiden Speicher sind auf eine Logikstufe 50 gegeben, die ein Logikglied 51 und eine Anzeige 52 enthält. Ueber einen Ausgang 53 kann das Signal der Logikstufe beispielsweise auf die Steuer- und Anzeigevorrichtung 10 zur Regelung der Tropfengeschwindigkeit gegeben werden.

Die Ausgangssignale der beiden Komparatoren 33 bzw. 43 sind auf eine Diskriminatorstufe 60 gegeben, die eingangsseitig ein erstes Logikglied 61 enthält, dessen Ausgangssignal einen Zähler 62 zurücksetzt und startet sowie ein weiteres Logikglied 63, auf das ein Zählerausgang und der Ausgang der Logikstufe 50 gegeben sind. Der Ausgang dieses Logikgliedes 63 steuert beispielsweise eine Fehler- oder Alarmvorrichtung 70.

Der Zähler 62 der Diskriminatorstufe 60 erhält seine Taktimpulse über einen Taktgeber 80. Weiterhin ist das Hall-Element 90 dargestellt, dessen Ausgangssignal auf den Speicher 44 geschaltet ist. Die dargestellte Vorrichtung zur Registrierung der Tropfen kann kontinuierlich betrieben werden. Eine vorteilhafte und insbesondere für den ambulanten Betrieb derartiger Infusionsgeräte günstige Verminderung des Energieverbrauches lässt sich dadurch erzielen, dass über den Taktgeber die Lichtquellen, die Detektoren und auch das Hall-Element getaktet werden.

Gemäss der vorliegenden Erfindung besteht die Vorrichtung zur Registrierung der Tropfen aus zwei Tropfenfühlern, von denen jeder seine eigene Detektorschaltung mit entsprechender Elektronik für die Tropfendetektion aufweist. Die beiden Tropfenfühler können vorteilhafterweise unterschiedliche, insbesondere einstellbare Empfindlichkeit besitzen. Beispielsweise lässt sich das über die Komparatoren 33 oder 43 einstellen. Die Ausgangssignale der beiden Tropfenfühler 30 bzw. 40 gelangen auf die Logikstufe 50, in der über das Logikglied 51 festgestellt wird, ob beide Tropfenfühler einen Tropfen detektiert haben. In diesem Fall wird über das Ausgangssignal des Logikgliedes 51 beispielsweise die Anzeige 52 gesetzt. Ueber die Speicher 34 bzw. 44 wird sichergestellt, dass ein durch die beiden Tropfenfühler 30 bzw. 40 zeitlich nacheinander registrierter Tropfen das Logikglied derart ansteuern kann, dass wirklich eine Tropfenanzeige erfolgt.

Die Komparatorausgangssignale der Tropfenfühler sind weiterhin auf die Diskriminatorstufe 60 gegeben. Bereits durch ein Diskriminatorausgangssignal wird der Zähler 62 rückgesetzt und gestartet. Beim Erreichen einer bestimmten Zählrate gibt der Zähler an das Logikglied 63 ein Signal ab. Hat inzwischen auch der zweite Tropfenfühler einen Tropfen detektiert, so dass eine Änderung des Ausgangssignales der Logikstufe 50 stattgefunden hat, so wird die Fehler- bzw. Alarmvorrichtung nicht aktiviert.

Ungeachtet dessen, ob der Tropfen von beiden Tropfenfühlern oder nur von einem detektiert wurde, zählt der Zähler 62 bis zu einem weiteren Zählerstand weiter und setzt dann die beiden Speicher 34 bzw. 44 in den Ausgangszustand zurück. Als Speicher können in diesem Fall einfache Speicher-Flipflops (Latches) verwendet werden. Die Aufgabe der Diskriminatorstufe 60 ist es also, ein Warnsignal zu erzeugen, wenn einer der Tropfenfühler, beispielsweise aufgrund von Nebel in der Tropfenkammer, Spritzern an der Wand der Tropfenkammer oder aufgrund irgendwelcher anderer Störungen einen Tropfen nicht mehr detektiert. Macht man insbesondere die beiden Tropfenfühler unterschiedlich empfindlich, so wird beispielsweise bei einer langsam zunehmenden Verschmutzung der Tropfenkammerwand ab einem bestimmten Grenzwert ein Fehler- oder Alarmsignal ausgelöst. Dabei funktioniert ein Tropfenfühler immer noch einwandfrei, so dass die Funktionskontrolle sicher durchführbar ist.

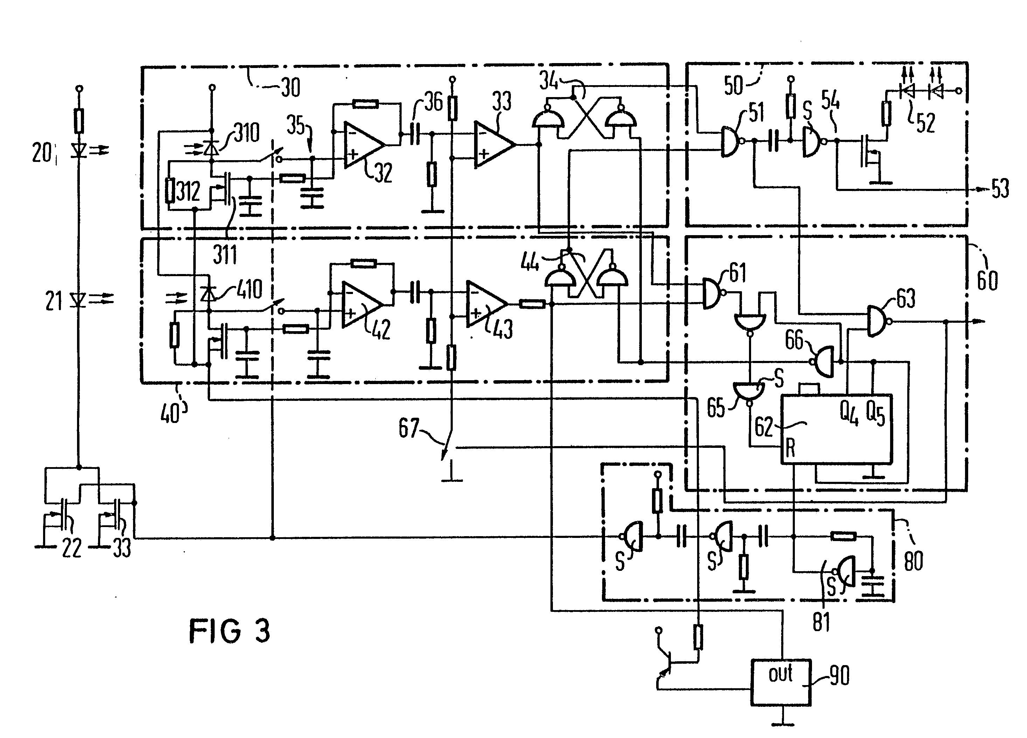

In Fig. 3 ist im folgenden ein spezielles Ausführungsbeispiel der verwendeten Elektronik für die erfindungsgemässe Vorrichtung beschrieben. Gleiche Teile sind dabei mit gleichen Bezugszeichen — wie in den vorangehenden Figuren — versehen.

Als Lichtquelle dienen zwei IR-LED 20, 21, die beispielsweise über Schalttransistoren 22, 23 mit einer Frequenz von annähernd 200 Hz und einer Impulslänge von 1,5 $\mu$s getaktet werden. Die Taktfrequenz erhält man über den Taktgeber 80, der einen ständig schwingenden Oszillator 81 und eine Reihe von Impulsformungskomponenten aufweist.

Da die Tropfenfühler 30 bzw. 40 abgesehen von evtl. unterschiedlichen Empfindlichkeiten identisch sind, wird im folgenden nur der Schaltungsaufbau des Tropfenfühlers 30 beschrieben. Als Lichtsensor dient eine IR-Fotodiode 310, die zusammen mit einem MOS-Transistor 311 und einem Widerstand 312 einen Spannungsteiler bildet und synchron mit der Lichtquelle 20 getaktet wird. Dies geschieht ebenfalls über den Taktgeber 80. Die Taktfrequenz ist verständlicherweise die gleiche, die Dauer der Taktimpulse kann jedoch grösser gewählt werden, damit der Empfang der ausgesendeten Lichtimpulse in jedem Fall sichergestellt ist. Beispielsweise können die Taktimpulse eine Länge von 10 $\mu$s besitzen. Das Signal des gebildeten Spannungsteilers wird über ein « sample and hold »-Glied 35 auf den Verstärker 32 gegeben. Die nachfolgende Verarbeitung der empfangenen Signale wird dadurch unabhängig von deren Zeitdauer. Die Vorspannung des Verstärkers 32 in Gegenkopplung wird an das Gate des MOS-Transistors im Spannungsteiler zurückgeführt, wodurch eine automatische Offset-Justierung der Spannungsteilung erreicht wird.

Ueber einen Kondensator 36 wird das Ausgangssignal des Verstärkers 32 AC-mässig auf einen Komparator 33 geschaltet, der beispielsweise die positiven Flanken des Signales erfasst. Das Ausgangssignal dieses Komparators 33 wird auf ein Speicher-Flipflop 34 (Latch) gegeben, das dadurch gesetzt wird. Dieses Latch wird durch die Diskriminatorstufe 60 zurückgesetzt. Sobald ein Komparatorausgangssignal vorliegt und damit ein Speicher-Flipflop gesetzt wird oder auch wenn beide gesetzt werden, wird der Zähler 62 der Diskriminatorstufe 60 über das Logikglied 61, ein NAND-Gatter 64, und gegebenenfalls weitere Logik- bzw. Invertierglieder zurückgesetzt und gestartet. Der Zähltakt wird von dem gleichen Taktgeber 80 an den Rechner abgegeben, der auch die Lichtquelle und die Lichtsensoren taktet. Die Zählfrequenz liegt — wie bereits erwähnt — bei ca. 200 Hz. Wenn der Zähler im vorliegenden Ausführungsbeispiel 16 Taktimpulse gezählt hat was ca. hat, was ca. 80 ms entspricht, wird über den Zähleraugang $Q_4$ ein Signal auf das Logikglied 63, ebenfalls ein NAND-Gatter gegeben, das ein Fehlersignal abgibt, wenn nicht beide Speicher-Flipflops 34 bzw. 44 gesetzt sind, d. h. ein Tropfen von beiden Tropfenfühlern detektiert wurde.

Unabhängig davon, welcher Fall vorliegt, zählt der Zähler weitere 80 ms, um danach anzuhalten und in diesem Falle über ein weiteres Invertierglied 66 die beiden Speicher-Flipflops 34 bzw. 44 zurückzusetzen. Wenn ein Fehlerfall vorliegt, d. h. wenn der Ausgang des NAND-Gatters 63 seinen Zustand geändert hat, dann wird im vorliegenden Ausführungsbeispiel über einen Schalter 67 die Vergleichsspannung an den Komparatoren 33 bzw. 43 derart geändert, dass diese weiteren Tropfen nicht detektieren können. Dieser Zustand kann bis zum Erreichen des Zählerendstandes beibehalten werden, so dass das Fehlersignal für eine genügend lange Zeit zur sicheren Ansteuerung von Fehlergliedern zur Verfügung steht.

Die Ausgangssignale beider Latches 34, 44 sind auf das Logikglied 51 der Logikstufe 50 — wiederum ein NAND-Gatter — geschaltet. Sind beide Latches gesetzt, d. h. haben beide Tropfenfühler den Tropfen detektiert, so ändert sich der Ausgangszustand des Logikgliedes 51. Dieses Signal gelangt auf das Logikglied 63 und gleichzeitig auf einen Impulsgeber 54, der die Tropfenanzeige 52, z. B. Leuchtdioden, ansteuert.

Das Hall-Element 90 wird — wie bereits eingangs ausgeführt — dazu verwendet, über einen an der Tropfenkammer befestigten und hier nicht dargestellten Magneten die Lage der Tropfenkammer in der Haltevorrichtung 3 gemäss Fig. 1 anzuzeigen. Selbstverständlich ist es ebenfalls möglich, den Magneten in der Haltevorrichtung und das Hall-Element an der Tropfenkammer anzubringen. Auch das Hall-Element wird getaktet, um den Energieverbrauch weiter zu senken. Wird die Tropfenkammer aus der Haltevorrichtung 3 herausgenommen oder befindet sie sich dort nicht in der vorgeschriebenen Lage, so ändert sich der Abstand zwischen Magnet und Hall-Element, wodurch das Hall-Element ein Ausgangssignal erzeugt, das auf ein Speicher-Flipflop gegeben wird und dieses setzt. Das führt automatisch zu einem Fehlersignal am Ausgang des NAND-Gatters 63. Auf diese Art und Weise ist es praktisch ohne zusätzlichen Schaltungsaufwand möglich, neben der Funktionskontrolle der Vorrichtung zur Detektierung der Tropfen unter Verwendung der gleichen Elektronik eine Lagekontrolle der Tropfenkammer in der Haltevorrichtung durchzuführen.

Die erfindungsgemässe Vorrichtung kann innerhalb der gesamten Rechenzeit von im vorliegendem Beispiel ca. 160 ms jeweils nur einen Tropfen nachweisen. Das hat den grossen Vorteil, dass durch diesen Tropfen beispielsweise von der Oberfläche den Infusionsflüssigkeit in der Tropfenkammer ausgehende Spritzer in diesem Zeitintervall keine Fehldetektionen hervorrufen können. Andererseits ist die Zeit kurz genug, um bei üblichen Durchflussgeschwindigkeiten der Infusionsflüssigkeit alle Tropfen zu detektieren. Sollte im Extremfall eine höhere Tropfenfrequenz erforderlich sein, so detektiert die Vorrichtung dann nur beispielsweise jeden zweiten Tropfen. Die Funktionskontrolle der Vorrichtung ist jedoch unverändert sichergestellt.

**Patentansprüche**

1. Vorrichtung zur Registrierung der Tropfen in einem Infusionsgerät mit einer an einen Vorratsbehälter (1) für Infusionsflüssigkeit anschliessbaren Tropfenkammer (2), von der die Infusionsflüssigkeit über eine Leitung (7) einem Patienten zuführbar ist, und mit einem im Bereich der Tropfenkammer (2) vorgesehenen Tropfenfühler, der mindestens eine Lichtquelle (20, 21) und zumindest zwei in Tropfrichtung versetzt angeordnete Lichtsensoren (31, 41 ; 310, 410) aufweist, dadurch gekennzeichnet, dass jedem Lichtsensor (31, 41 ; 310, 410) eine eigene Detektorschaltung (32, 33, 34) zugeordnet ist, dass die Ausgangssignale der Detektorschaltungen auf eine Logikstufe (50), die beim Vorliegen eines von mindestens einem Sensor detektierten Tropfens eine Tropfenanzeige (52) und/oder eine Steueranordnung für die Tropfengeschwindigkeit ansteuert, und auf eine Diskriminatorstufe (60), die, wenn innerhalb einer vorbestimmten Zeit nicht alle Detektorschaltungen einen Tropfen registriert haben, eine Fehlanzeige und/oder eine Alarmstufe (70) betätigt, geschaltet sind.

2. Vorrichtung zur Registrierung der Tropfen nach Anspruch 1, dadurch gekennzeichnet, dass die Tropfenfühler (30, 40) unterschiedliche Empfindlichkeit aufweisen.

3. Vorrichtung zur Registrierung der Tropfen nach Anspruch 1 oder 2, dadurch gekennzeichnet, dass als Lichtquellen mindestens zwei in Tropfrichtung versetzt angeordnete Leuchtdioden (20, 21) vorgesehen sind.

4. Vorrichtung zur Registrierung der Tropfen nach einem der Ansprüche 1 bis 3, dadurch gekennzeichnet, dass der Sensor (310) zusammen mit einem Transistor (311), insbesondere einem MOS-Transistor, einen Spannungsteilen bildet.

5. Vorrichtung zur Registrierung der Tropfen nach Anspruch 4, dadurch gekennzeichnet, dass das von den Spannungsteilen abgegriffene Signal auf einen Verstärker (32) und gegebenfalls Impulsformer geschaltet ist.

6. Vorrichtung zur Registrierung der Tropfen nach Anspruch 4 oder 5, dadurch gekennzeichnet, dass die Vorspannung des Verstärkers in Rückkopplung auf den Steuereingang des Transistors (311) geschaltet ist.

7. Vorrichtung zur Registrierung der Tropfen nach Anspruch 5 oder 6, dadurch gekennzeichnet, dass die Verstärker (32) unterschiedliche Empfindlichkeit haben.

8. Vorrichtung zur Registrierung der Tropfen nach einem der Ansprüche 5 bis 7, dadurch gekennzeichnet, dass das Verstärkersignal wechselspannungsmässig auf einen Eingang eines Komparators (33) gegeben ist, an dessem anderen Eingang eine Vergleichsspannung anliegt.

9. Vorrichtung zur Registrierung der Tropfen nach Anspruch 8 dadurch gekennzeichnet, dass die an den anderen Eingang des Komparators (33) angelegte Vergleichsspannung für jeden Komparator unterschiedlich, insbesondere einstellbar ist.

10. Vorrichtung zur Registrierung der Tropfen nach Anspruch 8 oder 9, dadurch gekennzeichnet, dass das Ausgangssignal des Komparators (33) auf ein Speichen-Flipflop (34) (Latch) geschaltet ist, das über die Diskriminatorstufe (60) rücksetzbar und dessen Ausgang auf die Logikstufe (50) geschaltet ist.

11. Vorrichtung zur Registrierung der Tropfen nach einem der Ansprüche 1 bis 10, dadurch gekennzeichnet, dass die Logikstufe (50) ein logisches Verknüpfungsglied (51) aufweist, das, wenn mindestens ein Speicher-Flipflop gesetzt ist, umschaltet.

12. Vorrichtung zur Registrierung der Tropfen nach Anspruch 11, dadurch gekennzeichnet, dass über das Verknüpfungsglied (51) ein Impulsgeber (54) betätigtbar ist, der eine Tropfenanzeige (52) ansteuert.

13. Vorrichtung zur Registrierung der Tropfen nach einem der Ansprüche 1 bis 12, dadurch gekennzeichnet, dass die Diskriminatorstufe (60) einen getakteten Zähler (62) aufweist, der gegebenenfalls über Logik- und/oder Invertierglieder (61, 64, 65) durch ein oder mehrere Komparatorausgangssignale rückgesetzt und gestartet wird und der bei einer bestimmten, einstellbaren Zählerstellung ein erstes und bei einer bestimmten weiteren und höheren Zählerstellung ein zweites Ausgangssignal liefert, und dass das erste Ausgangssignal zusammen mit dem Ausgangssignal des logischen Verknüpfungsgliedes (51) der Logikstufe (50) auf ein weiteres logisches Verknüpfungsglied (63) gegeben ist, über das die Fehleranzeige und/oder die Alarmstufe betätigtbar ist, und das zweite Ausgangssignal die Speicher-Flipflops (34, 44) zurücksetzt.

14. Vorrichtung zur Registrierung der Tropfen nach einem der Ansprüche 1 bis 13, dadurch gekennzeichnet, dass die Lichtquellen und/oder Lichtsensoren synchron getaktet sind.

15. Vorrichtung zur Registrierung der Tropfen nach Anspruch 14, dadurch gekennzeichnet, dass zum Takten ein Oszillator (81) vorgesehen ist, der auch das Taktsignal für den Zähler (62) liefert.

16. Vorrichtung zur Registrierung der Tropfen nach einem der Ansprüche 1 bis 15, dadurch gekennzeichnet, dass eine Haltevorrichtung (3) für die Tropfenkammer (2) vorgesehen ist, die ein Hall-Element (90) aufweist, dass die Tropfenkammer (2) mit einem Magneten versehen ist und dass das Ausgangssignal des Hall-Elementes (90) auf ein Speicher-Flipflop (44) geschaltet ist.

17. Vorrichtung zur Registrierung der Tropfen nach Anspruch 16, dadurch gekennzeichnet, dass das Hall-Element (90) ebenfalls getaktet ist, insbesondere über den gleichen Taktgeber (80) wie Lichtquelle, Sensor und Zähler.

**Claims**

1. A drip recorder for an infusion apparatus comprising a drip chamber (2) for connection to an infusion liquid supply container (1), from which the infusion liquid can be supplied to a patient via a pipeline (7), a drip sensor arranged in the region of the drip chamber (2) and including at least one light source (20, 21) and at least two light sensors (31, 41 ; 310, 410) arranged in staggered fashion in the drip direction, characterised in that each light sensor (31, 41 ; 310, 410) is assigned its own detector circuit (32, 33, 34), that the output signals of the detector circuits are fed to a logic stage (50), which drives a drip display (52) and/or a control arrangement for the drip rate in the presence of a drip detected by at least one sensor, and are fed to a discriminator stage (60) which actuates a fault display and/or an alarm stage (70) when a drip has not been recorded by all the detector circuits within a predetermined interval of time.

2. A drip recorder as claimed in Claim 1, characterised in that the drip sensors (30, 40) exhibit different levels of sensitivity.

3. A drip recorder as claimed in Claims 1 or 2, characterised in that at least two luminescence diodes (20, 21) are provided as light sources, arranged in staggered fashion in the drip direction.

4. A drip recorder as claimed in one of Claims 1 to 3, characterised in that the sensor (310), together with a transistor (311), in particular a MOS-transistor, forms a voltage divider.

5. A drip recorder as claimed in Claim 4, characterised in that the signal tapped from the voltage dividers is fed to an amplifier (32), and to a pulse shaper where appropriate.

6. A drip recorder as claimed in Claims 4 or 5, characterised in that the bias voltage of the amplifier is fed-back to the control input of the transistor (311).

7. A drip recorder as claimed in Claims 5 or 6, characterised in that the amplifiers (32) exhibit different levels of sensitivity.

8. A drip recorder as claimed in one of Claims 5 to 7, characterised in that the amplifier signal is fed as an a. c. voltage to one input of a comparator (33) whose other input is connected to a comparison voltage.

9. A drip recorder as claimed in Claim 8, characterised in that different, adjustable comparison voltages are connected to the other input of each comparator (33).

10. A drip recorder as claimed in Claims 8 or 9, characterised in that the output signal of the comparator (33) is fed to a storage flip-flop (34) (latch) which can be reset via the discriminator stage (60) and whose output is connected to the logic stage (50).

11. A drip recorder as claimed in one of Claims 1 to 10, characterised in that the logic stage (50) includes a logic-linking element (51) which switches-over when at least one storage flip-flop is set.

12. A drip recorder as claimed in Claim 11, characterised in that a pulse generator (54) which drives a drip display (52) can be actuated via the logic-linking element (51).

13. A drip recorder as claimed in one of Claims 1 to 12, characterised in that the discriminator stage (60) includes a clock-controlled counter (62) which is reset and started via logic and/or inverter components (61, 64, 65) by one or more than one comparator output signal where appropriate, and supplies a first output signal at a specified, adjustable count and supplies a second output signal at a specified further, higher count, and that the first output signal is fed together with the output signal from the logic-linking element (51) of the logic-stage (50) to a further logic-linking element (63) via which the fault display and/or alarm stage can be actuated and that the second output signal resets the storage flip-flop (34, 44).

14. A drip recorder as claimed in one of Claims 1 to 13, characterised in that the light sources and/or light sensors are clock-controlled in synchronism.

15. A drip recorder as claimed in Claim 14, characterised in that for the clock-control an oscillator (81) is provided which also supplies the clock signal for the counter (62).

16. A drip recorder as claimed in one of Claims 1 to 15, characterised in that a holder (3) comprising a Hall-element (90) is provided for the drip chamber (2), that the drip chamber (2) is provided with a magnet, and that the output signal of the Hall-element (90) is fed to a storage flip-flop (44).

17. A drip recorder as claimed in Claim 16, characterised in that the Hall-element (90) is clock-controlled, in particular via the same clock generator (80) as the light source, sensor and counter.

## Revendications

1. Dispositif pour enregistrer les gouttes dans un appareil de perfusion, avec une chambre à gouttes (2) susceptible d'être reliée à un réservoir de réserve (1) pour le liquide de perfusion et à partir de laquelle le liquide de perfusion peut être dispensé, par l'intermédiaire d'un tuyau (7), à un patient, et avec un détecteur de gouttes prévu au niveau de la chambre à gouttes (2) et comportant au moins une source lumineuse (20, 21) et avec au moins deux capteurs de lumière (31, 41 ; 310, 410) disposés avec décalage dans la direction de passage des gouttes, caractérisé par le fait qu'à chaque capteur de lumière (31, 41 ; 310, 410) est associé un circuit de détection (32, 33, 34) qui lui est propre, que les signaux de sortie des circuits de détection sont appliqués à un étage logique (50) qui commande, lors de la présence d'une goutte détectée par au moins un capteur, un dispositif d'affichage des gouttes (52) et/ou un dispositif de commande pour la vitesse des gouttes, ainsi qu'à un étage discriminateur (60) qui, si pendant un intervalle de temps prédéterminé tous les circuits de détection n'ont pas enregistré une goutte, commande l'affichage d'un défaut et/ou un étage d'alarme (70).

2. Dispositif pour enregistrer les gouttes selon la revendication 1, caractérisé par le fait que le détecteur de gouttes (30, 40) présente des sensibilités différentes.

3. Dispositif pour enregistrer les gouttes selon la revendication 1 ou 2, caractérisé par le fait que l'on prévoit, en tant que sources lumineuses, au moins deux diodes électroluminescentes (20, 21) qui sont disposées avec décalage mutuel dans la direction du passage des gouttes.

4. Dispositif pour enregistrer les gouttes selon l'une des revendications 1 à 3, caractérisé par le fait que le capteur (310) forme, avec un transistor (311), en particulier un transistor MOS, un diviseur de tension.

5. Dispositif pour enregistrer les gouttes selon la revendication 4, caractérisé par le fait que le signal prélevé par les parties de la tension, est appliqué à un amplificateur (32), et éventuellement à un formateur d'impulsions.

6. Dispositif pour enregistrer les gouttes selon la revendication 4 ou 5, caractérisé par le fait que la tension de polarisation de l'amplificateur est appliquée, avec rétroaction, à l'entrée de commande du transistor (311).

7. Dispositif pour enregistrer les gouttes selon la revendication 5 ou 6, caractérisé par le fait que les amplificateurs (32) possèdent des sensibilités différentes.

8. Dispositif pour enregistrer les gouttes selon l'une des revendications 5 à 7, caractérisé par le fait que le signal de l'amplificateur est appliqué, en tant que tension alternative, à une entrée d'un comparateur (33) à l'autre entrée duquel est appliquée une tension de comparaison.

9. Dispositif pour enregistrer les gouttes selon la revendication 8, caractérisé par le fait que la tension de comparaison appliquée à ladite autre entrée du comparateur (33) est différente pour chaque comparateur, et est en particulier réglable.

10. Dispositif pour enregistrer les gouttes selon la revendication 8 ou 9, caractérisé par le fait que le signal de sortie du comparateur (33) est appliqué à une mémoire à multivibrateur bistable (34) qui, par l'intermédiaire de l'étage discriminateur (60) peut être remis dans son état initial et dont la sortie est appliquée à l'étage logique (50).

11. Dispositif pour enregistrer les gouttes selon l'une des revendications 1 à 10, caractérisé par le fait que l'étage logique (50) comporte un circuit combinatoire logique qui est commuté dans le cas où au moins une mémoire à multivibrateur bistable est commandée.

12. Dispositif pour enregistrer les gouttes selon la revendication 11, caractérisé par le fait que par l'intermédiaire du circuit combinatoire (151), un générateur d'impulsions (54) est susceptible d'être commandé, lequel générateur d'impulsions attaque un dispositif d'affichage des gouttes (52).

13. Dispositif pour enregistrer les gouttes selon l'une des revendications 1 à 12, caractérisé par le fait que l'étage discriminateur (60) comporte un compteur (62) cadencé, qui est, éventuellement par l'intermédiaire d'éléments logiques et/ou inverseurs (61, 64, 65), susceptible d'être mis dans son état initial ou d'être démarré par un ou plusieurs signaux de sortie du comparateur et qui, pour une position de comptage déterminée et réglable, fournit un premier et, pour une autre position de comptage plus élevée, un second signal de sortie, et que le premier signal de sortie, avec le second signal de sortie du circuit combinatoire logique (51) de l'étage logique (50), est appliqué à un second circuit combinatoire logique (63) par l'intermédiaire duquel est susceptible d'être commandé le dispositif d'affichage de l'erreur et/ou l'étage d'alarme, alors que le second signal de sortie remet dans son état initial la mémoire à multivibrateur bistable (33, 34).

14. Dispositif pour enregistrer les gouttes selon l'une des revendications 1 à 13, caractérisé par le fait que les sources lumineuses et/ou les capteurs de lumière sont cadencés de façon synchrone.

15. Dispositif pour enregistrer les gouttes selon la revendication 14, caractérisé par le fait que pour la cadence, il est prévu un oscillateur (81) qui fournit également le signal de cadence pour le compteur (62).

16. Dispositif pour enregistrer les gouttes selon l'une des revendications 1 à 15, caractérisé par le fait qu'il est prévu pour la chambre à gouttes (2) un dispositif de support (3) qui comporte un élément de Hall (90), que la chambre à gouttes (2) est pourvue d'un aimant, et que le signal de sortie de l'élément de Hall (90), est appliqué à une mémoire à multivibrateur bistable (44).

17. Dispositif pour enregistrer les gouttes selon la revendication 16, caractérisé par le fait que l'élément de Hall (90) est également cadencé, en particulier à l'aide du même générateur de cadence (14) qui est utilisé pour la source lumineuse, le capteur de lumière et le compteur.

# FIG 1

FIG 2

FIG 3